(19) **Europäisches Patentamt European Patent Office Office européen des brevets**

(11) **EP 1 734 369 B1**

(12) **EUROPÄISCHE PATENTSCHRIFT**

(45) Veröffentlichungstag und Bekanntmachung des Hinweises auf die Patenterteilung:
**18.03.2009 Patentblatt 2009/12**

(51) Int Cl.:
*G01N 33/96* (2006.01)    *G01N 33/86* (2006.01)

(21) Anmeldenummer: **06010455.1**

(22) Anmeldetag: **20.05.2006**

(54) **Verfahren zur Standardisierung von Gerinnungstesten**

Procedure for the standardisation of coagulation tests

Procédure de normalisation des essais de coagulation

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.06.2005 DE 102005028018**

(43) Veröffentlichungstag der Anmeldung:
**20.12.2006 Patentblatt 2006/51**

(73) Patentinhaber: **Siemens Healthcare Diagnostics Products GmbH**
**35041 Marburg (DE)**

(72) Erfinder:
• **Wilkens, Matthias, Dr.**
**35037 Marburg (DE)**
• **Zander, Norbert, Dr.**
**35039 Marburg (DE)**

(56) Entgegenhaltungen:
**EP-A- 0 562 425        EP-A- 0 743 521**
**US-A- 5 670 329**

• **ADCOCK DM AND DUFF S: "Enhanced standardization of the International Normalized Ratio through the use of plasma calibrants: a concise review" BLOOD COAGULATION AND FIBRINOLYSIS, Bd. 11, Nr. 7, Oktober 2000 (2000-10), Seiten 583-590, XP008066244 ISSN: 0957-5235**
• **BRIEN WILLIAM F ET AL: "In-house calibration of the International Sensitivity Index or calibration curve for determination of the international normalized ratio." ARCHIVES OF PATHOLOGY & LABORATORY MEDICINE, Bd. 128, Nr. 3, März 2004 (2004-03), Seiten 308-312, XP002389073 ISSN: 0363-0153**
• **TALSTAD INGEBRIGT: "Why is the standardization of prothrombin a problem?" HAEMOSTASIS, Bd. 30, Nr. 5, September 2000 (2000-09), Seiten 258-267, XP008066258 ISSN: 0301-0147**

EP 1 734 369 B1

## Beschreibung

Die vorliegende Erfindung liegt auf dem Gebiet der Gerinnungsdiagnostik und betrifft ein Verfahren zur Standardisierung von in vitro-Gerinnungstesten.

Gerinnungsteste ermöglichen die Messung der Aktivität eines einzelnen oder mehrerer Gerinnungsfaktoren durch die Messung der Fibrinbildungsgeschwindigkeit in vitro, d. h. außerhalb des menschlichen oder tierischen Körpers (Koagulometrie). Das primäre Ergebnis dieser Teste ist eine Gerinnungszeit, die üblicherweise vom Zeitpunkt der Zugabe eines Aktivators und/oder von $Ca^{2+}$-Ionen zur Probe bzw. zum Probengemisch bis zur Bildung eines fassbaren Fibringerinnsels in Sekunden gemessen wird. Eine Gerinnungszeit ist also ein Maß für das hämostatische Potenzial, die Gerinnungsfähigkeit, einer Probe, wobei die Einflüsse aller gerinnungsfördernden und gerinnungshemmenden Faktoren und Substanzen, die in der Probe enthalten sind und die durch den jeweiligen Test erfasst werden, zum Tragen kommen.

Gerinnungszeiten können durch manuelle oder automatische Methoden bestimmt werden. Bei der automatischen Bestimmung ist die Messung einer mechanischen oder einer optischen Eigenschaft des Proben-/Reagenzgemisches, z. B. der Viskosität oder Trübung, sehr verbreitet. In allen Fällen automatischer Messung wird eine Eigenschaft des Proben-/Reagenzgemisches kontinuierlich gemessen, und aus der zeitabhängigen Änderung der Eigenschaft kann mit Hilfe von Auswerteverfahren die Gerinnungszeit als Endpunkt bestimmt werden.

Typische Beispiele für derartige Gerinnungsteste sind die Prothrombinzeit (PT), die auch Quick-Test oder Thromboplastinzeit genannt wird, die aktivierte partielle Thromboplastinzeit (APTT), die Thrombinzeit (TT), die Batroxobinzeit (BT) oder die Ecarinzeit (ECT). Diese Teste und ihre Varianten werden gewöhnlich zum Screening auf Defekte in einem Teilbereich des Gerinnungssystems verwendet (Screeningteste, Globalteste, Suchteste) oder zur Aktivitätsmessung von Einzelfaktoren. [Übersicht in Barthels, M. und von Depka, M.: Das Gerinnungskompendium, Georg Thieme Verlag Stuttgart, 2003]. Zu den Defekten des Gerinnungssystems, die eine Blutungsneigung oder eine Thromboseneigung zur Folge haben können, gehören zum Beispiel (a) sehr geringe oder sehr hohe Konzentrationen oder Aktivitäten von Gerinnungsfaktoren, (b) Mutanten von Gerinnungsfaktoren, (c) sehr geringe oder sehr hohe Konzentrationen oder Aktivitäten von Inhibitoren, (d) Mutanten von Inhibitoren oder (e) Antikörper gegen Elemente des Gerinnungssystems.

Das Patentdokument US 5,670,329 beschreibt beispielsweise ein Verfahren zur quantitativen Bestimmung des Einzelfaktors Fibrinogen anhand der Thrombinzeit einer Probe. Dazu werden die Gerinnungszeiten von Kalibratoren mit bekanntem Fibrinogengehalt in dem gleichen lokalen Testsystem gemessen, wie die zu bestimmende Probe, und die gemessene Gerinnungszeit der Probe wird mit Hilfe einer Kalibrationskurve, die aus der Zuordnung der bekannten Fibrinogengehalte und der im Testsystem gemessenen Gerinnungszeiten der Kalibratoren erstellt wurde, in den Fibrinogengehalt der Probe umgewandelt. Das in US 5,670,329 beschriebene Verfahren kann auch als Screeningtest für abnormale Fibrinogenkonzentrationen eingesetzt werden, sofern das Probenmaterial unverdünnt verwendet wird.

Im klinischen Alltag werden Screeningteste primär zur Diagnose hämorrhagischer oder thrombophiler Diathesen eingesetzt wie auch zur Überwachung von Therapien mit Medikamenten, die das Gerinnungssystem beeinflussen. Die Bestimmung der APTT zum Beispiel dient einerseits dem Screening auf Defekte des Teiles der Gerinnungskaskade, der über den sogenannten intrinsischen Weg gestartet wird und im gemeinsamen Weg aufgeht, und welcher aus den Gerinnungsfaktoren FVIII, FIX, FXI, FXII, Praekallikrein, HMW Kininogen, FV, FX, FII und Fibrinogen besteht. Ein APTT-Ergebnis oberhalb des Normalbereiches, d. h. eine verlängerte Gerinnungszeit kann auf einen Defekt eines oder mehrerer dieser Faktoren, zum Beispiel auf einen FVIII-Defekt, auch bekannt als Hämophilie A, hindeuten. Andererseits reagiert die APTT auch empfindlich auf die Anwesenheit von Antikoagulanzien, wie z. B. von Heparin, und wird daher auch zur Überwachung von Heparintherapien verwendet.

Für die medizinische Beurteilung eines Gerinnungstestergebnisses wird das Testergebnis einer Patientenprobe mit einem sogenannten Referenzbereich (auch Normalbereich genannt) verglichen. Der Referenzbereich eines Gerinnungstests wird festgelegt, indem der Test an einer großen Anzahl offensichtlich gesunder Personen (bevorzugt ≥ 20) durchgeführt wird. Manche der Gesunden werden niedrige Testwerte haben, manche etwas höhere, die meisten Werte werden um einen Mittelwert streuen. Zeichnet man die Testergebnisse auf der X-Achse und die Anzahl der Personen auf der Y-Achse auf, erhält man im Idealfall eine Normalverteilung. Mit statistischen Methoden bestimmt man gewöhnlicherweise die untere und die obere Referenzgrenze, innerhalb derer 90 % aller Gesunden liegen. In der Regel umfasst der Referenzbereich (Normalbereich) also 90 % der Werte, die bei gesunden Referenzpersonen gemessen wurden. Unter Umständen ist es notwendig, für einen Test verschiedene, zum Beispiel alters- oder geschlechtsabhängige Referenzbereiche festzulegen.

Weicht das Ergebnis einer Patientenprobe vom Referenzbereich ab, kann dies für eine Störung des hämostatischen Gleichgewichts sprechen. Eine Abweichung vom Referenzbereich der APTT in Richtung längerer Gerinnungszeiten kann zum Beispiel einen Faktor VIII-Defekt anzeigen; eine Abweichung vom Referenzbereich der PT in Richtung kürzerer Gerinnungszeiten kann zum Beispiel einen erhöhten Faktor II-Spiegel anzeigen, beispielsweise verursacht durch eine Mutation in der Promotorregion des Faktor II-Gens, die zu einer vermehrten Faktor II-Synthese führt.

Bei der Überwachung von Therapien mit Medikamenten, die das Gerinnungssystem beeinflussen, wie z. B. der Therapie mit Antikoagulanzien, geht es um die Einstellung des Gerinnungssystems des Patienten auf eine bestimmte Zielgröße. Hierbei wird die Medikation so lange verändert, bis das Testergebnis der Patientenprobe in einem definierten, sogenannten therapeutischen Bereich liegt. Der therapeutische Bereich wird in der Regel in umfangreichen klinischen Studien ermittelt. Hierbei wird ein Maximum an therapeutischer Effizienz und ein Minimum an unerwünschten Nebenwirkungen in Beziehung zu einem bestimmten Wertebereich eines diagnostischen Testergebnisses bzw. zu einem bestimmten Konzentrationsbereich des Medikaments gesetzt.

Die medikamentöse Hemmung der Gerinnungsfähigkeit durch die Verabreichung von Antikoagulanzien, wie z. B. Heparin, spielt eine wichtige Rolle bei der Prophylaxe oder Therapie thromboembolischer Ereignisse. Die Effizienz der Antikoagulation wird durch die Zahl unerwünschter thromboembolischer Ereignisse in der Klinik gemessen. Unerwünschte Nebenwirkungen der Antikoagulation können Blutungskomplikationen sein. Der therapeutische Wertebereich eines Tests, der sich zur Steuerung einer antikoagulatorischen Therapie eignet, ist also der Messwertebereich des Tests, in welchem in einer klinischen Studie ein Minimum an thromboembolischen sowie an Blutungskomplikationen beobachtet wurde.

Es ist bekannt, dass die Testergebnisse hämostaseologischer Teste einer Varianz unterliegen und daher nicht ohne weiteres vergleichbar sind. Diese Varianz beruht u. a. auf der Verwendung verschiedener Reagenzien und auf Unterschieden bei der technischen Ermittlung der Gerinnungszeit. So steht z. B. für die Ermittlung der Prothrombinzeit (PT) eine Vielzahl von Gewebethromboplastinen zur Verfügung. Die PT ein und derselben Plasmaprobe kann sich je nach verwendetem Gewebethromboplastin bis zu einem Faktor von 2 unterscheiden. Selbst bei der Verwendung eines speziellen Gewebethromboplastins variiert die Gerinnungszeit einer Probe je nach Hersteller, je nach Charge eines Herstellers oder unter Umständen sogar je nach Flasche einer einzigen Charge. Weiterhin übt bei der automatischen Bestimmung einer Gerinnungszeit das Messgerät selbst und die Art der Gerinnseldetektion einen Einfluss auf die Gerinnungszeit aus. Die Messung ein und derselben Probe mit demselben Gewebethromboplastin an unterschiedlichen Geräten liefert unterschiedliche Testergebnisse.

In der Praxis erstellt jedes Labor für einen verwendeten Gerinnungstest lokale, laborinterne Referenz- und therapeutische Bereiche. Um dem behandelnden Arzt eine Beurteilung eines Testergebnisses zu ermöglichen, müssen neben dem eigentlichen Testergebnis auch Informationen zu den lokalen Referenzbereichen bzw. den therapeutischen Bereichen eines Testes mitgeliefert werden.

Im Stand der Technik sind verschiedene Standardisierungsverfahren bekannt, die es zum Ziel haben, reagenz-, hersteller-, chargen- und geräteabhängige Unterschiede zwischen den Testergebnissen auszugleichen. Die Standardisierung von Testergebnissen bzw. die Vergleichbarkeit von Testergebnissen ist aus verschiedenen Gründen von besonderem Vorteil. Einerseits wird es dadurch möglich, Testergebnisse, die in verschiedenen Laboratorien im Rahmen klinischer Studien ermittelt wurden, global miteinander zu vergleichen, andererseits wird die Interpretation von Patienten-Testergebnissen wesentlich vereinfacht.

Gängige Standardisierungsverfahren beruhen auf Normalplasmaverdünnungen (Standardisierung in % der Norm, z. B. beim Quick-Test), Verhältnisbildung (Standardisierung in Ratio) sowie normalisierter Verhältnisbildung (Internationale Normalisierte Ratio INR beim Prothrombintest).

Bei der Verwendung von Normalplasmaverdünnungen wird zunächst ein Referenzplasmapool (Normalplasmapool) aus dem Plasma von in der Regel mindestens 20 offensichtlich gesunden Spendern hergestellt. Die Aktivität bzw. das hämostatische Potenzial dieses Standards wird mit 100 % der Norm definiert. Verdünnungen dieses Standardplasmas in einer geeigneten Matrix werden hergestellt. Zum Beispiel wird ein Teil Normalplasmapool mit zwei Teilen eines geeigneten Puffers vermischt (1:2 Verdünnung) und mit einer Aktivität von 33,33 % der Norm definiert. Durch Messung des Normalplasmapools und der Reihe von Verdünnungen dieses Pools mit Hilfe des zu standardisierenden Testsystems wird eine Referenzkurve (Kalibrationskurve) erstellt, indem die definierten Aktivitäten in % der Norm den gemessenen Rohwerten zugeordnet werden. Für Patientenproben kann dann in dem standardisierten Testsystem der Rohwert gemessen werden und schließlich anhand der Referenzkurve in einen kalibrierten Wert bzw. ein standardisiertes Testergebnis, nämlich in % der Norm umgerechnet werden.

Bei den Standardisierungsverfahren, die auf Verhältnisbildung beruhen, sind prinzipiell zwei Vorgehensweisen zu unterscheiden. Im ersten Fall werden für jede Probe zwei verschiedene Teste durchgeführt und die Ergebnisse zueinander in Verhältnis (Ratio) gesetzt. Für diese Ratio können Referenzbereiche und therapeutische Bereiche bestimmt werden. Im zweiten Fall werden im gleichen Testsystem das Ergebnis einer Patientenprobe und das Ergebnis eines Normalplasmapools ermittelt und die Ratio gebildet. Ein solches Verfahren existiert z. B. für die Standardisierung der Prothrombinzeit (PT), wobei die Prothrombin-Ratio (PR) das Verhältnis der PT einer Patientenprobe zur PT eines Normalplasmas angibt ($PR = PT_{Patient} / PT_{Normal}$).

Zur weiteren Standardisierung der Prothrombin-Ratio (PR) wurde von der Weltgesundheitsorganisation (WHO) ein Referenzthromboplastin geschaffen, an das mit anderen Thromboplastinen ermittelte Prothrombin-Ratios angeglichen werden können, so dass die ermittelte PR in eine International Normierte Ratio (INR) umgerechnet werden kann. Als Maß für die Empfindlichkeit eines Thromboplastinreagenzes im Vergleich zu der Empfindlichkeit des WHO-Thromboplastinreagenzes wurde ein Korrekturfaktor eingeführt, der Internationale Sensitivitätsindex (ISI), so dass die INR gemäß der Formel $INR = PR^{ISI}$ berechnet werden kann. Für das WHO-Thromboplastinreagenz ist der ISI-Wert mit 1,0 definiert, so dass INR = PR ist. Für alle anderen Thromboplastine werden von den Herstellern ISI-Werte mitgeteilt.

Trotz dieser Anstrengungen ist bei einer Reihe von Gerinnungstesten, z. B. bei der aktivierten partiellen Thromboplastinzeit (APTT), der Thrombinzeit (TT), der Batroxobinzeit (BT) oder der Ecarinzeit (ECT) bis heute keine überzeugende Standardisierung möglich. Zwar gab es in der Vergangenheit Bemühungen, die APTT analog zur Prothrombinzeit durch Verhältnisbildung zwischen der APTT einer Patientenprobe und der APTT eines Normalplasmas zu standardisieren, und es wurde versucht, den therapeutischen Bereich für die APTT-Ratio auf 1,5-2,5 zu definieren. Allerdings hat sich das Verfahren nie durchgesetzt, da auch die standardisierten Werte (APTT-Ratio) eine zu große Varianz aufwiesen, um eine akzeptable Vergleichbarkeit von Messergebnissen zu gewährleisten [Brill-Edwards, P. et al. (1993) Establishing a therapeutic range for heparin therapy. Ann Intern Med. 119 (2), 104-109]. Alternativ wurde in Analogie zur Berechnung der INR für die Prothrombinzeit versucht, eine Standardisierung der APTT über ein Referenzreagenz zu etablieren [Reed, S. V. et al. (1994) An attempt to standardize the APTT for heparin monitoring, using the P.T. ISI/INR system of calibration. Results of a 13 centre study. Thromb Res. 74 (5), 515-522; van der Velde, E. A. & Poller, L. (1995) The APTT monitoring of heparin - the ISTH/ICSH collaborative study. Thromb Haemost. 73(1), 73-81.]. Die Ergebnisse waren jedoch weitgehend entttäuschend, da ebenfalls keine akzeptable Vergleichbarkeit der Messergebnisse erzielt werden konnte.

Besonders problematisch ist die fehlende Standardisierung der Gerinnungsteste bei der Überwachung von Therapien mit Antikoagulanzien. Die Problematik soll hier exemplarisch am Beispiel der Überwachung einer Heparinbehandlung mit Hilfe der APTT dargestellt werden.

Heparin hemmt sofort und konzentrationsabhängig das Gerinnungssystem. Verwendet werden unfraktioniertes Heparin (UFH) sowie niedermolekulare Heparine (LMWH). Eine Heparintherapie erfolgt bei thromboembolischen Ereignissen, die bereits eingetreten sind oder akut vorliegen. Eine Heparinprophylaxe soll das Eintreten thrombolischer Ereignisse verhindern. Als therapeutische Bereiche sowohl für die Heparintherapie wie auch für die Heparinprophylaxe werden üblicherweise die einzustellenden Heparinspiegel (in IU/ml) angegeben. So gilt für die Heparintherapie ein therapeutischer Bereich von 0,3 - 0,7 IU/ml und für die Heparinprophylaxe ein therapeutischer Bereich von etwa 0,05 - 0,25 IU/ml.

Die Therapie und Prophylaxe mit Heparin, inbesondere mit unfraktioniertem Heparin, kann einerseits mit Methoden überwacht werden, die den Heparinspiegel direkt messen, andererseits auch mit Gerinnungstesten, die den Einfluss des Heparins auf die Gerinnungsfähigkeit des Blutes messen.

Die direkte Bestimmung des Heparinspiegels erfolgt z. B. über sogenannte Faktor Xabasierende oder Anti-Xa-Methoden, bei denen die Faktor Xa-inhibierende Aktivität einer Patientenprobe gemessen wird. Dieser Test kann mit Hilfe von Internationalen Heparin Standards standardisiert werden und liefert demnach ein standardisiertes, vergleichbares Ergebnis in Form eines Konzentrationswertes in Internationalen Einheiten (Units) pro Milliliter (IU/ml). Diese Methode zur Bestimmung des Heparinspiegels hat sich jedoch in der Routinediagnostik nicht durchgesetzt, da das erforderliche Testsystem kostspielig ist und in vielen Laboren nicht zur Verfügung steht.

Alternativ wird die Überwachung einer Heparintherapie heutzutage über die Messung der APTT durchgeführt. Bei der Überwachung einer Heparintherapie mit Hilfe der APTT geht es darum, die Heparingabe so zu steuern, dass sich ein APTT-Ergebnis im therapeutischen Bereich ergibt. Da es aufgrund der testsystemabhängigen Varianz der APTT-Werte keinen standardisierten therapeutischen Bereich für APTT-Werte gibt, ist es notwendig für jedes Testsystem den therapeutischen Bereich zu bestimmen. Für die Bestimmung des therapeutischen Bereichs des APTT-Tests für unfraktioniertes Heparin gibt es grundsätzlich zwei Möglichkeiten [Nelson, D. E. (1999) Current considerations in the use of the APTT in monitoring unfractionated heparin. Clin Lab Sci. 12 (6), 359-64; Olson, J. D. et al. (1998) College of American Pathologists Conference XXXI on laboratory monitoring of anticoagulant therapy: laboratory monitoring of unfractionated heparin therapy. Arch Pathol Lab Med. 122 (9), 782-98]:

Bei dem sogenannten ex vivo-Verfahren werden Plasmaproben einer Gruppe heparinisierter Patienten genommen. Von jeder Probe wird der Heparinspiegel in IU/ml mit einem Faktor Xa-Test bestimmt und die APTT mit einem Testsystem bestimmt. Anschließend wird eine lineare Regression zwischen den beiden Datensätzen (Heparinspiegel in IU/ml und APTT in s) durchgeführt. Der therapeutische Bereich im APTT-Testsystem ist der Wertebereich, der dem Heparinkonzentrationsbereich von 0,3 - 0,7 IU/ml entspricht (siehe auch Beispiel 1). Das ex vivo-Verfahren ist verhältnismäßig umständlich und auch kostspielig. Es setzt eine größere Anzahl von verfügbaren Patienten- und Normalproben sowie die Verfügbarkeit einer Faktor Xabasierenden Methode zur Bestimmung des Heparinspiegels voraus.

Aufgrund der vorgenannten Nachteile der ex vivo-Methode hat sich in der Laborpraxis ein sogenanntes in vitro-Verfahren etabliert. Bei diesem Verfahren zur Bestimmung des therapeutischen Bereichs eines APTT-Tests wird ein Normalplasmapool mit standardisierten Mengen Heparin versetzt ("gespikt") und die APTT der so hergestellten Proben gemessen. Unbedingt zu beachten ist allerdings, dass eine in vitro-Probe (z. B. Heparin gespiktes Normalplasma) verglichen mit einer ex vivo-Probe mit gleichem Heparinspiegel zu anderen Gerinnungszeiten führen kann. Zur Bestimmung des therapeutischen Bereichs eines APTT-Tests mit Hilfe eines in vitro-Verfahrens müssen daher andere Grenzen für den Heparingehalt der Kalibrationsproben berücksichtigt werden. In Publikationen wird einem ex vivo-Heparinkonzentrationsbereich von 0,3 - 0,7 IU/ml ein in vitro-Bereich von 0,2 - 0,4 IU/ml zugeordnet. Das in vitro-Verfahren zur Bestimmung des therapeutischen APTT-Bereiches für die Heparintherapie ist ein Annäherungsverfahren mit begrenzter Aussagekraft.

Bislang liefern Teste zur Bestimmung der APTT, der TT, der BT und der ECT eine Gerinnungszeit, die allein in Sekunden ausgewertet wird, wobei das Ergebnis stets vom lokalen Testsystem, d. h. von Reagenz, Reagenziencharge, Varianz von Flasche zu Flasche, Testdurchführung, Automatisierung, Messgerät, Messverfahren und Auswerteverfahren abhängt. Die globale Vergleichbarkeit der Testergebnisse ist somit kaum gegeben.

Der vorliegenden Erfindung lag also die Aufgabe zugrunde, ein in vitro-Verfahren bereitzustellen, dass die Standardisierung von Gerinnungstesten ermöglicht und damit die globale Vergleichbarkeit von Testwerten, die mit Hilfe unterschiedlicher (lokaler) Testsysteme ermittelt wurden, gewährleistet.

Die erfindungsgemäße Lösung besteht in der Bereitstellung der in den Ansprüchen beschriebenen Gegenstände und Verfahren.

Unter dem Begriff "Gerinnungszeit" ist im Sinne der vorliegenden Erfindung ein Testergebnis eines in vitro-Gerinnungstests zu verstehen, bei welchem die Zeitspanne ab der Zugabe eines Aktivatorreagenzes und/oder von $Ca^{2+}$-Ionen zu einer Probe oder einem Probengemisch bis zur fassbaren Bildung eines Fibringerinnsels in Sekunden [s] gemessen wird oder, sofern ein chromogenes Substrat verwendet wird, die Zeitspanne ab der Zugabe eines Aktivatorreagenzes und/oder $Ca^{2+}$-Ionen zur Probe bis zur Erreichung einer definierten Absorptionsänderungsgeschwindigkeit. Die Verwendung eines chromogenen Substrats ist z. B. bei Methoden zur Bestimmung der PT bekannt (siehe z. B. EP 14 039 A1).

Das erfindungsgemäße Verfahren ermöglicht die Bestimmung einer standardisierten Gerinnungszeit einer Probe, indem die gemessene Gerinnungszeit der Probe (primäres Testergebnis, Ist-Wert) anhand einer Kalibrationskurve (Referenzkurve) in eine standardisierte Gerinnungszeit (standardisiertes Testergebnis, Soll-Wert) umgewandelt wird.

Besonders bevorzugt wird ein Verfahren zur Bestimmung einer standardisierten Gerinnungszeit aus der Gruppe Prothrombinzeit (PT), aktivierte partielle Thromboplastinzeit (APTT), Thrombinzeit (TT), Batroxobinzeit (BT) und Ecarinzeit (ECT).

Zur Erstellung der Kalibrationskurve ist die Verfügbarkeit von mindestens zwei Kalibratoren notwendig, wobei für jeden Kalibrator vorab eine diskrete Standardgerinnungszeiten in Sekunden [s] festgelegt wurde. Die Anzahl der Kalibratoren, die eingesetzt werden kann, ist nach oben nicht begrenzt.

Die Festlegung der Standardgerinnungszeit eines Kalibrators kann auf verschiedene Arten erfolgen. Eine Möglichkeit ist es, die Standardgerinnungszeit eines Kalibrators in einem einzigen Durchlauf eines Gerinnungstests zu bestimmen, wobei dieser Testdurchlauf unter den definierten Bedingungen eines spezifischen, lokalen Gerinnungstestsystems erfolgt, d. h. die Standardgerinnungszeit wird unter Verwendung eines spezifischen Aktivatorreagenzes einer spezifischen Reagenziencharge an einem spezifischen Messgerät gemessen. Möglich ist zum Beispiel die Verwendung einer spezifischen APTT-Reagenzcharge zur Bestimmung der APTT eines Kalibrators und die Zuordnung der unter diesen spezifischen Bedingungen gemessenen APTT als APTT-Standardgerinnungszeit. Eine andere Möglichkeit ist es, die Standardgerinnungszeit eines Kalibrators durch Mittelung einer Mehrzahl von Testergebnissen eines Gerinnungstests festzulegen. Bevorzugterweise werden dazu unterschiedliche Aktivatorreagenzchargen verwendet. Es können aber auch alternative Aktivatorreagenztypen verwendet werden, wie z. B. unterschiedliche APTT-Aktivatoren (Kaolin oder Ellagsäure oder Silica etc.). Aus den in den einzelnen Testdurchläufen gemessenen Gerinnungszeiten wird dann z. B. der Mittelwert gebildet und dem Kalibrator als Standardgerinnungszeit zugeordnet. Bevorzugterweise werden die Standardgerinnungszeiten aller Kalibratoren, die zur Erstellung einer Referenzkurve verwendet werden sollen, auf dieselbe Art und Weise festgelegt und zugeordnet.

Bevorzugterweise wird zur Erstellung der Referenzkurve ein Satz (Kit) von Kalibratoren verwendet, die sich durch unterschiedliche hämostatische Potenziale und damit durch unterschiedliche Standardgerinnungszeiten auszeichnen. Ein anderer bevorzugter Kit enthält einen einzigen Kalibrator (Stammkalibrator) mit einer festgelegten, definierten Standardgerinnungszeit, der mit verschiedenen Volumina einer Verdünnungsflüssigkeit, z. B. einer Pufferlösung, verdünnt wird, wodurch eine Reihe von Kalibratoren hergestellt werden kann, die sich ebenfalls durch unterschiedliche hämostatische Potenziale auszeichnen. Bevorzugterweise ist die Verdünnungsflüssigkeit zusammen mit dem Stammkalibrator in einem erfindungsgemäßen Kit enthalten.

Die Erstellung der Kalibrationskurve erfolgt, indem die Gerinnungszeiten der Kalibratoren in dem gleichen lokalen Testsystem bestimmt werden wie die Gerinnungszeit einer zu bestimmenden Probe. Durch die Zuordnung der vorab bestimmten Standardgerinnungszeiten der Kalibratoren zu den dazugehörigen gemessenen Gerinnungszeiten und Interpolation bzw. Extrapolation wird eine Referenzkurve erstellt, wobei z. B. auf der x-Achse die Standardgerinnungszeit (Sollwert) und auf der y-Achse die gemessene Gerinnungszeit (Istwert) aufgetragen sind. Die gemessene Gerinnungszeit einer Probe wird anhand der Referenzkurve in eine standardisierte Gerinnungszeit umgewandelt.

Besonders bevorzugt werden Kalibratoren verwendet, die über die gleiche Matrix verfügen wie die Probe, deren Gerinnungszeit standardisiert werden soll. Das vorliegende Verfahren eignet sich besonders zur Standardisierung der Gerinnungszeiten von Plasmaproben, humanen oder tierischen Ursprungs, die mit Citrat versetzt sein können. Aus diesem Grund sind Kalibratoren zu bevorzugen, die ebenfalls auf einer Plasmabasis beruhen, bevorzugt auf Basis eines Plasmapools, wobei es sich dabei z. B. auch um Mischungen von humanem mit nicht-humanem Plasma handeln kann. Durch Mischen von humanem Plasma mit unterschiedlichen Anteilen von z. B. Kaninchen- und / oder bovinem Plasma und Messung der Gerinnungszeit eines solchen Plasmagemisches kann der Fachmann Kalibratoren zur Verwendung im erfindungsgemäßen Verfahren herstellen. Die Plasmakalibratoren können zusätzlich Substanzen enthalten, die bei der Herstellung von Plasmakalibratoren bzw. -kontrollen üblicherweise verwendet werden. Dazu gehören beispielsweise Puffersubstanzen wie z. B. TRIS oder HEPES, antikoagulierende Substanzen wie Citrat und Stabilisatoren, wie z. B. Dextran und Konservierungsmittel wie z. B. Natriumazid. Zur Verwendung in dem erfindungsgemäßen Verfahren können die Kalibratoren in flüssigem, gefrorenem oder gefriergetrocknetem Zustand bereitgestellt werden.

Eine besondere Ausführungsform des erfindungsgemäßen Verfahrens betrifft die Bestimmung einer standardisierten Gerinnungszeit einer Probe, die eine Substanz enthält, die das Gerinnungssystem beeinflusst. Bei derartigen Substanzen kann es sich um gerinnungshemmende Substanzen handeln, die einem Patienten im Zuge einer Antikoagulationstherapie verabreicht werden bzw. die für den Einsatz in einer Antikoagulationstherapie im Rahmen einer klinischen Prüfung oder in einer vergleichbaren Forschungsarbeit getestet werden. Dabei kann es sich z. B. um solche antikoagulatorisch wirksamen Medikamente oder Substanzen handeln wie Heparine, umfassend unfraktionierte, hochmolekulare Heparine (UFH), fraktionierte, niedermolekulare Heparine (NMH), halb- oder vollsynthetische Heparinoide (wie z. B. Danaparoid oder Orgaran®) oder Pentasaccharide, wie Fondaparinux; Vitamin K-Antagonisten, wie z. B. Cumarinderivate, oder direkte Thrombinhibitoren, wie Hirudin, Argatroban oder Melagatran. Weiterhin kann es sich um natürliche oder synthetische Faktor Xa-Inhibitoren handeln, wobei bei vielen natürlichen Faktor Xa-Inhibitoren auch rekombinante Varianten verwendbar sind. Beispiele für natürliche Faktor Xa-Inhibitoren, die im übrigen häufig aus dem Speichel hämatophager Tiere isoliert werden, sind Antistasin, ein Polypeptid aus dem mexikanischen Blutegel Haementeria officinalis, das Tick Anticoagulant Peptide, ein Polypeptid aus der Weichzecke Ornithodoros moubata, Yagin, ein Polypeptid aus dem Blutegel Hirudo medicinalis oder Draculin aus der Vampirfledermaus Desmodus rotundus. Die synthetischen Faktor Xa-Inhibitoren gehören verschiedenen Klassen an und werden unterschieden in Diamidino- und Bisbasische FXa-Inhibitoren, Mono-Benzamidin FXa-Inhibitoren und Nicht-Benzamidin FXa-Inhibitoren. Bei der Bestimmung der Gerinnungszeit von Proben antikoagulierter Patienten geht es u. a. darum festzustellen, ob die Antikoagulanzkonzentration im Blut bzw. Plasma des Patienten im therapeutischen Bereich liegt.

Für die Standardisierung der Gerinnungszeit solcher antikoagulierter Proben empfiehlt sich die Verwendung eines Kalibrators, der eine definierte Menge einer gerinnungshemmenden Substanz enthält, die in vitro eine gerinnungshemmende Funktion aufweist. Für die Standardisierung der Gerinnungszeit von Proben Heparin-behandelter Patienten beispielsweise empfiehlt sich die Verwendung eines Kalibrators, der mit einer definierten Menge Heparin gespikt wurde. Bevorzugterweise weist ein solcher Kalibrator eine Heparinkonzentration zwischen 0,1 und 1,0 IU/ml auf.

Die folgenden Ausführungsbeispiele dienen der Veranschaulichung des erfindungsgemäßen Verfahrens und sind nicht als Einschränkung zu verstehen.

**Beispiele**

**Beispiel 1:**

**Bestimmung des therapeutischen Bereichs für Heparin in einem APTT-Testsystem (ex vivo-Methode)**

Es wurden Plasmaproben Heparin-behandelter Patienten und ein Normalplasmapool verwendet. Für jede Probe wurde der Heparinspiegel in IU/ml gegen einen geeigneten Heparinstandard in einem Faktor Xa-Test bestimmt (Berichrom® Heparin, Dade Behring Marburg GmbH, Marburg, Deutschland). Ausserdem wurde die APTT jeder Probe unter Verwendung eines Aktivatorreagenzes (Phospholipide und Ellagsäure, Dade® Actin® FSL Reagenz, Dade Behring Marburg GmbH, Marburg, Deutschland) derselben Charge bestimmt. Die Ergebnisse dieser Messungen sind in Tabelle 1 aufgeführt.

**Tabelle 1**

|  | Heparin [IU/ml] | APTT [Sekunden] |  |  | Heparin [IU/ml] | APTT [Sekunden] |
|---|---|---|---|---|---|---|
| Normalpool | 0,00 | 29,4 |  |  |  |  |
| Patient | 0,60 | 75,9 |  | Patient | 0,24 | 66,4 |
| Patient | 0,60 | 75,9 |  | Patient | 0,25 | 49,1 |
| Patient | 0,01 | 42,5 |  | Patient | 0,27 | 55,3 |
| Patient | 0,02 | 57,4 |  | Patient | 0,27 | 25,8 |

(fortgesetzt)

| | Heparin [IU/ml] | APTT [Sekunden] | | | Heparin [IU/ml] | APTT [Sekunden] |
|---|---|---|---|---|---|---|
| Patient | 0,02 | 34,3 | | Patient | 0,31 | 70,6 |
| Patient | 0,04 | 36,2 | | Patient | 0,31 | 41,0 |
| Patient | 0,05 | 33,9 | | Patient | 0,32 | 57,5 |
| Patient | 0,05 | 34,4 | | Patient | 0,32 | 37,3 |
| Patient | 0,05 | 58,1 | | Patient | 0,33 | 55,3 |
| Patient | 0,05 | 34,5 | | Patient | 0,34 | 40,1 |
| Patient | 0,06 | 49,6 | | Patient | 0,34 | 51,1 |
| Patient | 0,06 | 27,1 | | Patient | 0,34 | 72,4 |
| Patient | 0,06 | 45,5 | | Patient | 0,36 | 64,8 |
| Patient | 0,07 | 27,1 | | Patient | 0,36 | 51,3 |
| Patient | 0,07 | 58,8 | | Patient | 0,37 | 75,1 |
| Patient | 0,07 | 84,2 | | Patient | 0,38 | 53,7 |
| Patient | 0,08 | 36,4 | | Patient | 0,39 | 70,6 |
| Patient | 0,10 | 46,5 | | Patient | 0,40 | 51,4 |
| Patient | 0,11 | 48,1 | | Patient | 0,41 | 72,3 |
| Patient | 0,12 | 52,3 | | Patient | 0,43 | 83,6 |
| Patient | 0,13 | 46,5 | | Patient | 0,43 | 70,2 |
| Patient | 0,17 | 39,5 | | Patient | 0,45 | 116,4 |
| Patient | 0,18 | 42,8 | | Patient | 0,49 | 63,9 |
| Patient | 0,19 | 62,8 | | Patient | 0,51 | 64,4 |
| Patient | 0,22 | 55,3 | | Patient | 0,52 | 69,3 |
| Patient | 0,22 | 67,9 | | Patient | 0,54 | 59,0 |
| Patient | 0,23 | 75,2 | | Patient | 0,69 | 73,9 |

Mit Hilfe der Software Microsoft® Excel Version 97 SR-2 (Microsoft Corporation, Redmond, USA) wurde eine lineare Regression der beiden Datensätze durchgeführt:

$$APTT\ [s] = a \times Heparin\ [IU/ml] + b$$

a = 58,47
b = 40,39

Anhand der Ausgleichsgeraden wurde der APTT-Wertebereich bestimmt, der dem allgemein bekannten therapeutischen Bereich des ex vivo-Heparinspiegels von 0,3 - 0,7 IU/ml entspricht:

**Tabelle 2**

| Heparin [IU/ml] | APTT [s] gemäß Regressionsgerade |
|---|---|
| 0,3 | 57,9 |
| 0,7 | 81,3 |

Demgemäß umfasste der therapeutische Bereich APTT-Werte von 57,9 bis 81,3 Sekunden.

**Beispiel 2:**

**Herstellung von Heparin-Kalibratoren und Festlegung ihrer diskreten Standard-APTTs für die Verwendung in einem erfindungsgemäßen Verfahren zur Standardisierung der APTT**

**Kalibrator 1: Normalplasmapool**

Plasma von 20 gesunden Spendern wurde in 3,2 % Natriumcitrat abgenommen, gepoolt, mit 50 mM HEPES-Puffer (pH 7,5) gepuffert, mit geeigneten Stabilisatoren stabilisiert, in 1 ml Aliquots abgefüllt und lyophilisiert. Zur Verwendung wird der Kalibrator mit 1 ml Aqua bidest. rekonstituiert.

**Kalibrator 2: Heparin-Plasmapool**

Zu einer geeigneten Stabilisatorlösung wurde eine definierte Menge an unfraktioniertem Heparin (Liquemin®, Roche Deutschland Holding GmbH, Grenzach-Wyhlen, Deutschland) zugesetzt, so dass eine Endkonzentration von 0,6 IU/ml erreicht wurde. Mit Hilfe eines mit einem internationalen Heparin-Standard kalibrierten Faktor Xa-Test (Berichrom® Heparin, Dade Behring Marburg GmbH, Marburg, Deutschland) wurde die Heparinaktivität der Heparinlösung an einem automatischen Gerinnungsmessgerät bestimmt. Der Faktor Xa-Test wurde mit einem internationalen Heparin-Standard kalibriert. Die Heparinlösung wurde in 1 ml Aliquots abgefüllt und lyophilisiert. Zur Verwendung wurde der Kalibrator mit 1 ml Normalplasmapool rekonstituiert.

Zur Bestimmung der Standard-APTT der beiden Kalibratoren wurde die APTT jedes Plasmas unter Verwendung eines Aktivatorreagenzes (Phospholipide und Ellagsäure, Dade® Actin® FSL Reagenz, Dade Behring Marburg GmbH, Marburg, Deutschland) derselben Charge am gleichen Gerinnungsmessgerät (BCS® Gerinnungsanalyzer, Dade Behring Marburg GmbH, Marburg, Deutschland) bestimmt. Es wurden folgende APTT-Werte bestimmt:

**Tabelle 3**

| | |
|---|---|
| Normalplasmapool | 29 s |
| Heparin-Plasmapool | 80 s |

Der APTT-Wert wurde dem entsprechenden Kalibrator nun als diskrete Standard-APTT (Sollwert) zugeordnet.

**Beispiel 3:**

**Bestimmung standardisierter APTT-Werte mit Hilfe des erfindungsgemäßen Verfahrens**

Die APTTs der beiden Kalibratoren (siehe Beispiel 2) und von Plasmaproben Heparin-behandelter Patienten wurden unter Verwendung eines Aktivatorreagenzes (Phospholipide und Ellagsäure, Dade® Actin® FSL Reagenz, Dade Behring Marburg GmbH, Marburg, Deutschland) einer beliebigen Charge an einem beliebigen Gerinnungsmessgerät bestimmt.

Für die beiden Kalibratoren ergaben sich aus der Zuordnung der vorab bestimmten Standard-APTT (Sollwert) und der parallel zu den Plasmaproben gemessenen APTT (Istwert) folgende Wertepaare:

**Tabelle 4**

| | Sollwert [s] | Istwert [s] |
|---|---|---|
| Normalplasmapool | 29 | 29,6 |
| Heparin-Plasmapool | 80 | 103,0 |

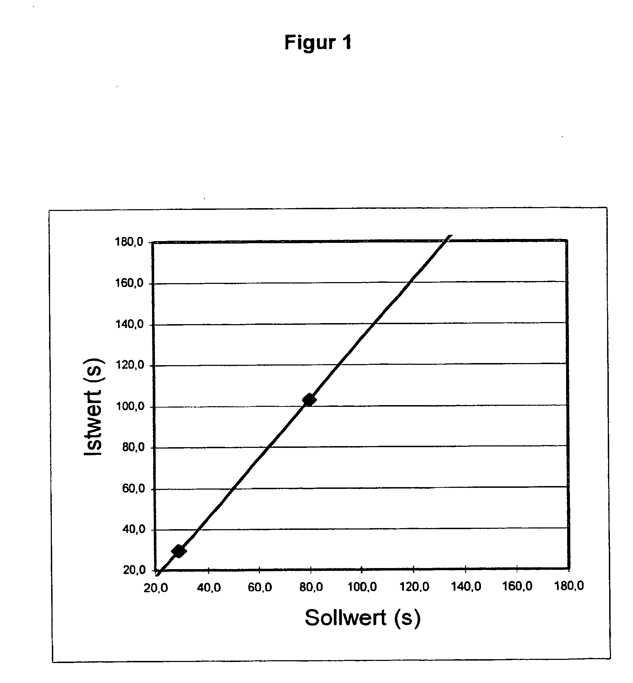

Per Extrapolation der beiden Wertepaare wurde eine Referenzkurve erstellt (Fig. 1).

Anhand dieser Referenzkurve wurden nun die gemessenen, primären APTT-Werte der Plasmaproben in standardisierte APTT-Werte umgewandelt (siehe Tab. 5). Die Erstellung der Referenzkurve wie auch die Umwandlung der Messwerte wurde automatisch vom Gerinnungsmessgerät durchgeführt.

**Tabelle 5**

| Gemessene APTT [s] | Standardisierte APTT [s] | Gemessene APTT [s] | Standardisierte APTT [s] |
|---|---|---|---|
| 29,6 | 29,0 | 93,8 | 73,6 |
| 103,0 | 80,0 | 59,8 | 50,0 |
| 103,0 | 80,0 | 66,1 | 54,4 |
| 50,2 | 43,3 | 27,9 | 27,8 |
| 67,2 | 55,1 | 84,7 | 67,3 |
| 44,8 | 39,6 | 44,9 | 39,6 |
| 46,6 | 40,8 | 80,2 | 64,2 |
| 41,2 | 37,1 | 41,5 | 37,3 |
| 35,3 | 33,0 | 63,9 | 52,8 |
| 58,3 | 48,9 | 41,2 | 37,1 |
| 35,3 | 33,0 | 58,8 | 49,3 |
| 59,7 | 49,9 | 100,4 | 78,2 |
| 28,8 | 28,4 | 82,6 | 65,8 |
| 51,4 | 44,1 | 64,1 | 53,0 |
| 28,7 | 28,4 | 83,7 | 66,6 |
| 68,2 | 55,8 | 69,3 | 56,6 |
| 79,0 | 63,3 | 90,0 | 71,0 |
| 42,0 | 37,6 | 66,1 | 54,4 |
| 57,8 | 48,6 | 94,7 | 74,2 |
| 57,6 | 48,5 | 106,6 | 82,5 |
| 63,9 | 52,8 | 88,2 | 69,7 |
| 65,4 | 53,9 | 122,4 | 93,5 |
| 47,8 | 41,6 | 83,5 | 66,5 |
| 59,9 | 50,1 | 74,1 | 59,9 |
| 81,8 | 65,3 | 108,5 | 83,8 |
| 63,6 | 52,6 | 106,7 | 82,6 |
| 76,5 | 61,6 | 98,5 | 76,9 |
| 105,9 | 82,0 | | |

**Beispiel 4:**

**Therapeutische Bereiche für verschiedene Chargen eines APTT-Reagenzes vor und nach Standardisierung der APTT**

Die APTTs der beiden Kalibratoren (siehe Beispiel 2) und von Plasmaproben Heparin-behandelter Patienten wurden unter Verwendung von vier unterschiedlichen Chargen eines Aktivatorreagenzes (Phospholipide und Ellagsäure, Dade® Actin® FSL Reagenz, Dade Behring Marburg GmbH, Marburg, Deutschland) an einem Gerinnungsmessgerät bestimmt. Gemäß Beispiel 3 wurde jeder chargenabhängige Messwert einer Probe anhand der chargenabhängigen Kalibrationskurve in einen standardisierten APTT-Wert umgewandelt. Die Heparinkonzentrationen der Patientenproben wurden in einem Faktor Xa-Test bestimmt (siehe Beispiel1).

Schließlich wurde sowohl für die gemessenen APTT-Werte als auch für die standardisierten APTT-Werte der APTT-Wertebereich bestimmt, der dem allgemein bekannten therapeutischen Bereich des ex vivo-Heparinspiegels von 0,3 - 0,7 IU/ml entspricht (siehe Beispiel 1).

**Tabelle 6**

| | Therapeutische Bereiche (0,3 - 0,7 IU/ml Heparin ex vivo) APTT-Wertebereiche in Sekunden | | | |
|---|---|---|---|---|
| | Charge 1 | Charge 2 | Charge 3 | Charge 4 |
| **vor** Standardisierung | 58-81 | 72-109 | 66-99 | 66-96 |
| **nach** Standardisierung | 66-96 | 64-95 | 67-99 | 66-96 |

Vor Standardisierung der APTT-Werte weichen die oberen Grenzen der therapeutischen Bereiche von Charge zu Charge um bis zu 35 % voneinander ab. Nach Standardisierung der APTT-Werte weichen die oberen Grenzen der therapeutischen Bereiche von Charge zu Charge nur noch um bis zu 4 % voneinander ab. Das erfindungsgemäße Verfahren ermöglicht eine Vereinheitlichung der therapeutischen Bereiche, wodurch die Ergebnisse verschiedener Testsysteme nach Standardisierung direkt miteinander vergleichbar sind.

**Patentansprüche**

1. Verfahren zur Bestimmung einer standardisierten Gerinnungszeit einer Probe **dadurch gekennzeichnet, dass**

   a) die Gerinnungszeiten von mindestens zwei Kalibratoren, für die vorab diskrete Standardgerinnungszeiten festgelegt wurden, in dem gleichen lokalen Testsystem gemessen werden wie die zu bestimmende Probe und
   b) die gemessene Gerinnungszeit der Probe mit Hilfe einer Kalibrationskurve, die aus der Zuordnung der vorab festgelegten Standardgerinnungszeiten und der in dem lokalen Testsystem gemessenen Gerinnungszeiten der Kalibratoren erstellt wurde, in eine standardisierte Gerinnungszeit umgewandelt wird.

2. Verfahren gemäß Anspruch 1 zur Bestimmung einer standardisierten Gerinnungszeit aus der Gruppe Prothrombinzeit (PT), aktivierte partielle Thromboplastinzeit (APTT), Thrombinzeit (TT), Batroxobinzeit (BT) und Ecarinzeit (ECT).

3. Verfahren nach den Ansprüchen 1 bis 2 zur Bestimmung einer standardisierten Gerinnungszeit einer Plasmaprobe.

4. Verfahren nach den Ansprüchen 1 bis 3 **dadurch gekennzeichnet, dass** Kalibratoren auf Plasmabasis verwendet werden.

5. Verfahren nach den Ansprüchen 1 bis 4 **dadurch gekennzeichnet, dass** Kalibratoren auf Basis eines Plasmapools verwendet werden.

6. Verfahren nach den Ansprüchen 1 bis 4 **dadurch gekennzeichnet, dass** Kalibratoren verwendet werden, die humanes Plasma oder eine Mischung von humanem und nicht-humanem Plasma enthalten.

7. Verfahren nach den Ansprüchen 1 bis 6 zur Bestimmung einer standardisierten Gerinnungszeit einer Probe, die eine Substanz enthält, die das Gerinnungssystem beeinflusst.

8. Verfahren nach Anspruch 7 zur Bestimmung einer standardisierten Gerinnungszeit einer Probe, die eine gerinnungshemmende Substanz enthält

9. Verfahren nach Anspruch 8 **dadurch gekennzeichnet, dass** die Probe eine Substanz aus der Gruppe Heparin, Heparinoide, Hirudin, Argatroban, Melagatran und natürliche oder synthetische Faktor Xa Inhibitoren enthält.

10. Verfahren nach den Ansprüchen 8 und 9 **dadurch gekennzeichnet, dass** mindestens ein Kalibrator eine definierte Menge einer gerinnungshemmenden Substanz enthält.

**11.** Verfahren nach Anspruch 10 **dadurch gekennzeichnet, dass** mindestens ein Kalibrator nach Zugabe von Heparin eine Heparinkonzentration von 0,1 bis 1,0 IU pro ml aufweist.

**12.** Verfahren nach den Ansprüchen 1 bis 11 **dadurch gekennzeichnet, dass** die Referenzkurve mit Hilfe eines Interpolations- oder Extrapolationsverfahrens erstellt wird.

**Claims**

**1.** A procedure for the determination of a standardized coagulation time of a sample, wherein

a) the coagulation times of at least two calibrators, for which discrete standard coagulation times have been previously established, are measured in the same local test system as the sample to be determined and
b) the measured coagulation time of the sample is converted to a standardized coagulation time with the aid of a calibration curve, which was plotted from the assignment of the previously established standard coagulation times and the coagulation times of the calibrators measured in the local test system.

**2.** The procedure as claimed in claim 1 for the determination of a standardized coagulation time from the group consisting of prothrombin time (PT), activated partial thromboplastin time (APTT), thrombin time (TT), batroxobin time (BT) and ecarin time (ECT).

**3.** The procedure as claimed in claims 1 and 2 for the determination of a standardized coagulation time of a plasma sample.

**4.** The procedure as claimed in claims 1 to 3, wherein calibrators based on plasma are used.

**5.** The procedure as claimed in claims 1 to 4, wherein calibrators based on a plasma pool are used.

**6.** The procedure as claimed in claims 1 to 4, wherein calibrators are used which contain human plasma or a mixture of human and nonhuman plasma.

**7.** The procedure as claimed in claims 1 to 6 for the determination of a standardized coagulation time of a sample which contains a substance which influences the coagulation system.

**8.** The procedure as claimed in claim 7 for the determination of a standardized coagulation time of a sample which contains an anticoagulatory substance.

**9.** The procedure as claimed in claim 8, wherein the sample contains a substance from the group consisting of heparin, heparinoids, hirudin, argatroban, melagatran and natural or synthetic factor Xa inhibitors.

**10.** The procedure as claimed in claims 8 and 9, wherein at least one calibrator contains a defined amount of an anticoagulatory substance.

**11.** The procedure as claimed in claim 10, wherein at least one calibrator, after addition of heparin, has a heparin concentration of 0.1 to 1.0 IU per ml.

**12.** The procedure as claimed in claims 1 to 11, wherein the reference curve is plotted with the aid of an interpolation or extrapolation procedure.

**Revendications**

**1.** Procédé pour la détermination d'un temps de coagulation normalisé d'un échantillon, **caractérisé en ce que**

a) on mesure dans le même système de test local que l'échantillon à déterminer les temps de coagulation d'au moins deux étalons pour lesquels des temps de coagulation distincts ont été préalablement déterminés, et
b) on convertit en un temps de coagulation normalisé le temps de coagulation mesuré de l'échantillon, à l'aide d'une courbe d'étalonnage qui a été construite à partir du classement des temps de coagulation normalisés, déterminés au préalable, et des temps de coagulation, mesurés dans le système de test local, des étalons.

2. Procédé selon la revendication 1 pour la détermination d'un temps de coagulation normalisé, choisi dans le groupe constitué par le temps de prothrombine (PT), le temps partiel de thromboplastine activé (APTT), le temps de thrombine (TT), le temps de batroxobine (BT) et le temps d'écarine (ECT).

3. Procédé selon les revendications 1 et 2 pour la détermination d'un temps de coagulation normalisé d'un échantillon de plasma.

4. Procédé selon les revendications 1 à 3, **caractérisé en ce qu'**on utilise des étalons à base de plasma.

5. Procédé selon les revendications 1 à 4, **caractérisé en ce qu'**on utilise des étalons à base d'un pool de plasmas.

6. Procédé selon les revendications 1 à 4, **caractérisé en ce qu'**on utilise des étalons qui contiennent du plasma humain ou un mélange de plasma humain et de plasma non humain.

7. Procédé selon les revendications 1 à 6 pour la détermination d'un temps de coagulation normalisé d'un échantillon qui contient une substance qui influe sur le système de coagulation.

8. Procédé selon la revendication 7 pour la détermination d'un temps de coagulation normalisé d'un échantillon qui contient une substance inhibant la coagulation.

9. Procédé selon la revendication 8, **caractérisé en ce que** l'échantillon contient une substance choisie dans le groupe constitué par l'héparine, les héparinoïdes, l'hirudine, l'argatroban, le mélagatran et des inhibiteurs naturels ou synthétiques du facteur Xa.

10. Procédé selon les revendications 8 et 9, **caractérisé en ce qu'**au moins un étalon contient une quantité définie d'une substance inhibant la coagulation.

11. Procédé selon la revendication 10, **caractérisé en ce qu'**au moins un étalon présente après addition d'héparine une concentration d'héparine de 0,1 à 1,0 UI par ml.

12. Procédé selon les revendications 1 à 11, **caractérisé en ce que** la courbe de référence est construite à l'aide d'un procédé d'interpolation ou d'extrapolation.

**Figur 1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5670329 A

- EP 14039 A1

**In der Beschreibung aufgeführte Nicht-Patentliteratur**

- **BARTHELS, M. ; VON DEPKA, M.** Das Gerinnungskompendium. Georg Thieme Verlag, 2003
- **BRILL-EDWARDS, P. et al.** Establishing a therapeutic range for heparin therapy. *Ann Intern Med.,* 1993, vol. 119 (2), 104-109
- **REED, S. V. et al.** An attempt to standardize the APTT for heparin monitoring, using the P.T. ISI/INR system of calibration. Results of a 13 centre study. *Thromb Res.,* 1994, vol. 74 (5), 515-522

- **VAN DER VELDE, E. A. ; POLLER, L.** The APTT monitoring of heparin - the ISTH/ICSH collaborative study. *Thromb Haemost.,* 1995, vol. 73 (1), 73-81
- **NELSON, D. E.** Current considerations in the use of the APTT in monitoring unfractionated heparin. *Clin Lab Sci.,* 1999, vol. 12 (6), 359-64
- **OLSON, J. D. et al.** College of American Pathologists Conference XXXI on laboratory monitoring of anticoagulant therapy: laboratory monitoring of unfractionated heparin therapy. *Arch Pathol Lab Med.,* 1998, vol. 122 (9), 782-98